# EUROPÄISCHE PATENTANMELDUNG

(11) **EP 0 761 221 A1**
(43) Veröffentlichungstag der Anmeldung: **12.03.1997**
(21) Anmeldenummer: 96112846.9
(22) Anmeldetag: 09.08.1996
(51) Int. Cl.: A61K 31/505

(54) **Verwendung von Orotsäure zur Herstellung eines Arzneimittels zur Prävention und Therapie des metabolischen Syndroms**

(30) Priorität: 17.08.1995 DE 19530298
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Verwendung von Orotsäure zur Prävention und Therapie des metabolischen Syndroms.

Das metabolische Syndrom,auch Syndrom X oder Wohlstandssyndrom genannt, besteht aus einer Gruppe von Symptomen, nämlich Glucoseintoleranz/Insulinresistenz, erhöhter Blutdruck, erhöhte Blutfette, Übergewicht (androide Fettverteilung), NIDDM-Diabetes (Non Insulin Dependent Diabetes; Diabetes mellitus Typ II).

## Beschreibung

Die Erfindung betrifft ein Produkt für die Prävention und Therapie des metabolischen Syndroms.

Das metabolische Syndrom, auch Syndrom X oder Wohlstandssyndrom genannt, besteht aus einer Gruppe von Symptomen, nämlich Glucoseintoleranz/Insulinresistenz, erhöhter Blutdruck, erhöhte Blutfette, Übergewicht (androide Fettverteilung), NIDDM-Diabetes (Non Insulin Dependent Diabetes; Diabetes mellitus Typ II). Das gehäufte gemeinsame Auftreten dieser Symptome wurde mit zunehmendem Wohlstand offenkundig und wird heute als Krankheitsentität akzeptiert. Als Syndrom wird eine Gruppe von gleichzeitig zusammen auftretenden Krankheitszeichen bezeichnet. Gekennzeichnet ist das metabolische Syndrom durch eine periphere Insulinresistenz, in deren Folge eine Hyperinsulinämie auftritt. Die Hyperinsulinämie ist hierbei für alle Facetten des Syndroms als mitursächlich anzusehen. Das Vollbild umfaßt die bauchbetonte, androide Fettsucht, die arterielle Hypertonie, die Dyslipoproteinämie, die Hyperurikämie und die gestörte Glukosetoleranz bis hin zum mannifesten Diabetes mellitus. Bestehen zwei oder mehr der genannten Faktoren, so ist das Vorliegen eines metabolischen Syndroms in Erwägung zu ziehen.

Es wird inzwischen vermutet, daß Bluthochdruck, Übergewicht, Diabetes mellitus sowie Fettstoffwechselstörungen eine gemeinsame metabolische Ursache haben, die darauf beruht, daß im Metabolismus dieser Patienten bestimmte Anomalien vorliegen, wahrscheinlich eine erhöhte Insulinresistenz.

Das metabolische Syndrom stellt infolge der Fülle atherogener Risikofaktoren sowohl für die präventive als auch die kurative Medizin eine Herausforderung dar.

Bisher wurde in diesem Zusammenhang jedes einzelne Symptom durch eine spezifische Therapie behandelt. So wurden gegen die Lipidstoffwechselstörungen Lipidsenker, gegen den Bluthochdruck blutdrucksenkende Mittel und gegen Diabetes mellitus orale Antidiabetika sowie Insulin eingesetzt. Im Falle des Übergewichtes wurde auf eine Diät zurückgegriffen, um das Gewicht des Patienten zurückzuführen.

Bei der Behandlung steht die Basistherapie mit Änderung der Lebensweise, Gewichtsreduktion, Einschränkung der Kochsalzzufuhr und Steigerung der körperlichen Aktivität im Vordergrund. Erst danach kommt die medikamentöse Therapie zum Einsatz. Dabei muß beachtet werden, daß die Beseitigung eines Risikofaktors nicht zur Verschlechterung oder zum Manifestwerden einer anderen Syndromfacette führt.

Eine Folgeerkrankung dieses metabolischen Syndroms ist auch die Arteriosklerose, die durch durchblutungssteigernde Mittel und durch Mittel zur Behandlung der koronaren Herzerkrankung oder zur Verbesserung des Gehirnstoffwechsels therapiert wird.

Zusammengefaßt bedeutet dies, daß bisher lediglich die einzelnen Symptome der unter dem Begriff metabolisches Syndrom zusammengefaßten Krankheiten behandelt wurden, daß jedoch ein Präparat zur Prävention oder Therapie des metabolischen Syndroms selbst noch fehlt.

Vor diesem Hintergrund ist es Aufgabe der vorliegenden Erfindung, ein Produkt zur Prävention und Therapie des metabolischen Syndroms bereitzustellen.

Erfindungsgemäß wird diese Aufgabe dadurch gelöst, daß Orotsäure zur Prävention und Therapie des metabolischen Syndroms verwendet wird.

Orotsäure wird bisher in Salzform als sogenannter Mineral- oder Elektrolyt-Schlepper eingesetzt und dient dazu, bestimmte Kationen in die Zelle zu transportieren. In diesem Zusammenhang ist es aus der DE-OS-24 10 181 beispielsweise bekannt, Lithiumorotat zu verwenden, um Natrium, das bei der Regulierung des Wasserhaushaltes eine Rolle spielt, in der Zelle durch Lithium zu ersetzen. Lithium wird hier in Form des Salzes der Orotsäure verwendet, da dieses den Transport des Lithiums in die Zelle bewirken kann.

Durch die Deplazierung des Natriums durch Lithium wird die Menge an gespeichertem Wasser reduziert. Dieser Effekt wird zur Therapie von Hypertonie, Arteriosklerose, Gefäß- und Gewebserkrankungen insbesondere im zerebralen Bereich sowie zur Dauer- und Schutzbehandlung von Migräne ausgenutzt.

In der Deutschen Apothekerzeitung Nr. 32, 10. August 1995, Seiten 52-53 ist zwar beschrieben, daß Magnesium und Orotsäure herzschützend wirken und zur Leistungssteigerung eingesetzt werden können, daß damit aber das gesamte Bündel der Symptome des metabolischen Syndroms behandelt werden kann, ist nicht ersichtlich.

Überraschenderweise konnte jetzt gefunden werden, daß die Orotsäure selbst im Zusammenhang mit dem metabolischen Syndrom präventiv und therapeutisch multipotent wirksam eingesetzt werden kann.

Die Orotsäure kann dabei direkt oder in Form eines ihrer Salze verwendet werden, wobei vorzugsweise Magnesiumorotat eingesetzt wird.

In einem Tierexperiment konnte nämlich gezeigt werden, daß Orotsäure arteriosklerotischen Gefäßveränderungen unter experimentellem Cholesterinstreß entgegenwirkt. Dabei zeigte sich, daß die Wirkung der reinen Orotsäure deutlich besser war als die von MgCl₂, so daß der Wirkeffekt bei Magnesiumorotat nicht primär auf Magnesium sondern auf die Orotsäure zurückzuführen ist.

Weiterhin wurde in diesem Tierexperiment gezeigt, daß Orotsäure blutdrucksenkend wirkt.

Schließlich konnte in einer Untersuchung an Triathleten gezeigt werden, daß nach einer Magnesiumorotat-Supplementierung das Insulin besser genutzt werden kann, was sich durch eine bessere Glukoseverwertung zeigt. Es zeigt sich eine Verbesserung der Leistungsfähigkeit, da durch die Verabreichung von Magnesiumorotat die Insulinresistenz reduziert wird.

In diesem Zusammenhang ergab sich weiter, daß die Harnsäurekonzentration durch die Magnesiumorotat-Supplementierung signifikant absank, was auf eine gesteigerte Harnsäure-Eliminierung in der Niere hindeutet.

In Einzelfallstudien (Kasuistiken) an Patienten konnte ferner gezeigt werden, daß Magnesiumorotat zur vorteilhaften Behandlung von Lipidstoffwechselstörungen, Diabetes mellitus (Insulinresistenz), Hyperurikämie sowie bei Bluthochdruck und zum Herz- und Gefäßschutz eingesetzt werden kann.

Aus diesen Ergebnissen läßt sich der überraschende Schluß ziehen, daß Orotsäure selbst sowohl zur Prävention als auch zur Therapie des metabolischen Syndroms verwendet werden kann, da es positive Auswirkungen auf alle Erkrankungen hat, die unter dem Begriff metabolisches Syndrom zusammengefaßt werden.

Dies ist als überraschend anzusehen und war in dieser Deutlichkeit und Ausprägung nicht zu erwarten.

In dem bereits erwähnten Tierexperiment konnte weiter gezeigt werden, daß die Verwendung von Magnesiumorotat gegenüber der Verwendung von reiner Orotsäure eine noch größere präventive und therapeutische Wirkung zeigt.

In einem Ausführungsbeispiel ist es dann bevorzugt, wenn eine Mischung aus Orotsäure und einer Magnesiumverbindung verwendet wird.

Hier ist von Vorteil, daß ein synergistischer Effekt auftritt, zum einen wird die Wirkung der Orotsäure ausgenutzt, wobei zusätzlich eine Magnesiumverbindung eingesetzt wird, um auch deren Wirkung mit zu verwenden.

Die Mischung besteht dabei vorzugsweise aus einem Salz der Orotsäure und einer Magnesiumverbindung, wobei auch eine Mischung aus Orotsäure und Magnesiumorotat verwendet werden kann.

Auch hier ist von Vorteil, daß neben der reinen Orotsäure Magnesium in dem herzustellenden Arzneimittel/Präparat/Produkt zu finden ist.

Das aus Orotsäure und/oder ihren Salzen hergestellte Arzneimittel/Produkt kann dabei in allen Darreichungsformen auftreten.

Die Erfindung wird nachfolgend anhand einiger Studien und im Zusammenhang mit den Figuren näher beschrieben und erläutert.

Es zeigen:
- Fig. 1: Indizes arteriosklerotischer Gefäßveränderungen unter experimentellem "Cholesterinstreß" bei Kaninchen; und
- Fig. 2: die Reduktion der in Wistar-Ratten experimentell induzierten, malignen Blutdrucksteigerung durch Magnesiumorotat.

### Beispiel 1: Untersuchung der Wirkung von Orotsäure und Magnesiumorotat in einem Arteriosklerose-Modell

In einer Versuchsreihe mit einem Arteriosklerose-Modell wurden Kaninchen über 112 Tage einer zweiprozentigen Cholesterindiät ausgesetzt und mit äquimolaren Mengen von Magnesiumchlorid, Orotsäure und Magnesiumorotat bzw. in der Kontrollgruppe überhaupt nicht weiter behandelt. Zusätzlich zu histologischen Untersuchungen der Koronaarterien sowie der Aorta femoralis und renalis wurde der Zustand der Aorta an jeweils mehreren Stellen auch planimetrisch-photographisch dokumentiert. Mittels einer computergestützten, digitalen Methode der Oberflächenmessung ("density slice"-Methode) erhält man hierbei dreidimensionale Bilder, mit deren Hilfe auch eine Quantifizierung der cholesterininduzierten Lumeneinengungen möglich ist.

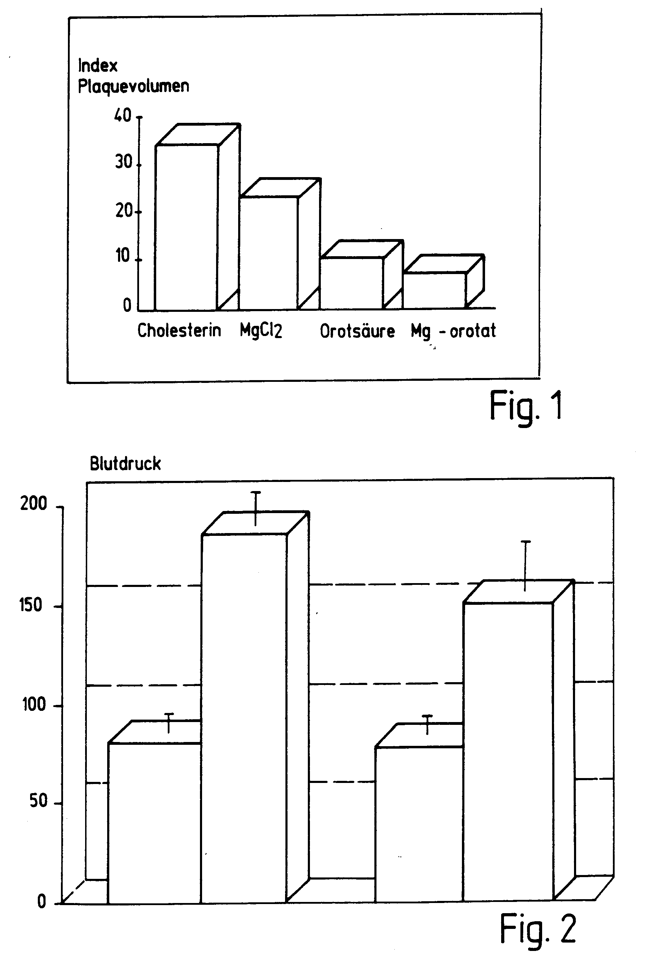

In Fig. 1 ist das Ergebnis dieser Untersuchung dargestellt. Es ist zu erkennen, daß die Gefäßverengungen in der unbehandelten Kontrollgruppe mit einem Index von 34,36 am ausgeprägtesten waren. Die Orotsäure wirkt mit einem Index von 9,77 deutlich besser den arteriosklerotischen Läsionen entgegen als Magnesiumchlorid (Index 22,63).

Als bester Schutz erwies sich jedoch Magnesiumorotat mit einem Index von 7,28.

Diese quantifizierten Ergebnisse wurden durch die licht- bzw. elektronenmikroskopischen Befunde sowie durch enzymhistochemische Untersuchungen bestätigt. Demnach verhütet Magnesiumorotat auch in den Koronarien die Genese sklerotischer Läsionen am besten.

Die lumeneinengende, durch die Cholesterinfütterung induzierte Proliferation von Schaumzellen war unter dieser Medikation weitaus am geringsten ausgeprägt.

Aus dieser Untersuchung ergibt sich, daß Orotsäure selbst arteriosklerotische Gefäßveränderungen entscheidend verhütet und daß diese Wirkung durch Zugabe von Magnesium weiter gesteigert werden kann.

### Beispiel 2: Untersuchung der Wirkung von Magnesiumorotat im Hypertonie-Tiermodell

In den Hypertonie-Tiermodellen nach Rojo-Ortega-Genest (ROG-Modell) und Lörincz-Goracz (LG-Modell) wird bei Wistar-Ratten die Aorta zwischen den beiden Renalarterien unterbunden bzw. die Niere in eine Gummimanschette fest eingepreßt, was einen malignen Hypertonus hervorruft, also eine Hypertonie mit konstanter Erhöhung des arteriellen diastolischen Blutdruckes auf > 120 mmHg.

Unter täglicher Zufuhr von 300 mg Magnesiumorotat waren weit weniger periarterielle Zellinfiltrationen und Herzmuskelzellausfälle zu beobachten als ohne Behandlung.

Das Ergebnis dieser Untersuchung ist in Fig. 2 dargestellt, wobei die dunklen Säulen jeweils den Beginn der ROG-Hypertonie anzeigen, während die hellen Säulen das Ende der ROG-Hypertonie darstellen. Links in Fig. 2 ist die ROG-Hypertonie ohne Behandlung gezeigt, während die beiden rechten Säulen den Verlauf der ROG-Hypertonie unter Zugabe von täglich 300 mg Magnesiumorotat wiedergibt.

Diese Therapie reduzierte die experimentiell induzierte, maligne Blutdrucksteigerung per se. Der Wirkeffekt des Magnesiumorotats ergibt sich dabei aus der Differenz zwischen den beiden hellen Säulen. Der Blutdruck betrug im ROG-Modell 152 mmHg systolisch gegenüber 186 mmHg bei unbehandelten Kontrolltieren nach Ausgangswerten von 79 bzw. 81 mmHg, war also bei den behandelten Tieren deutlich geringer ausgeprägt.

### Beispiel 3: Untersuchung der Wirkung von Magnesiumorotat auf den Energiestoffwechsel von Triathleten

In einer doppelblinden, randomisierten und placebokontrollierten Studie wurden 23 Triathleten einem 500 m Schwimm-, 20 km Fahrrad- und 5.000 m Lauftest nach einer vierwöchigen Magnesiumorotat-Supplementierung von 17 mmol/Tag unterworfen. Die Tests wurden ohne Pausen direkt hintereinander absolviert. Vor dem Test, zwischen den Disziplinen und am Ende wurde den Probanden Blut entnommen und auf Parameter des Energiestoffwechsels untersucht.

Vor dem Triathlon-Test waren die Glukosekonzentrationen beider Gruppen vergleichbar. Während des Tests stieg die Glukosekonzentration der Kontrollgruppe um 187 ± 34 %, die der Verumgruppe signifikant niedriger um 118 ± 45 % an. Gleichzeitig erhöhte sich die Insulinkonzentration der Kontrollgruppe durch den Streß auf 139 ± 101 %, in der Verumgruppe fiel die Insulinkonzentration im Vergleich zur Kontrolle dagegen signifikant auf 65 ± 43 % der Anfangswerte ab.

Diese streßabhängigen Veränderungen des Energiestoffwechsels deuten darauf hin, daß nach einer Magnesiumorotat-Supplementierung die Glukose aufgrund der besseren Insulinnutzung besser verwertet wird.

### Beispiel 4: Untersuchung der Wirkung von Magnesiumorotat auf den Harnsäurespiegel von Triathleten

In der Studie aus Beispiel 3 wurde zusätzlich der Harnsäurespiegel der Triathleten untersucht.

Es zeigte sich, daß durch die Magnesiumorotat-Supplementierung die Harnsäurekonzentration in der Verumgruppe signifikant auf 4,08 ± 1,38 mg/100 ml im Vergleich zur Kontrollgruppe absank, bei der die Konzentration 5,31 ± 0,75 mg/100 ml betrug.

Nach dem Triathlon stieg die Harnsäurekonzentration in der Verumgruppe auf 5,15 ± 1,23 mg/100 ml und in der Kontrollgruppe auf 7,67 ± 0,9 mg/100 ml an, so daß hier der Anstieg in der Verumgruppe signifikant geringer war als in der Kontrollgruppe.

Nach der Magnesiumorotat-Supplementierung scheint die Harnsäure-Eliminierung in der Niere sowohl in Ruhe als auch deutlich während physischem Streß gesteigert zu werden.

Die obigen Beispiele zeigen, daß Orotsäure für sich genommen bereits die einzelnen, unter dem Begriff metabolisches Syndrom zusammengefaßten Krankheiten und Erkrankungen deutlich therapiert und hier auch präventiv wirkt. Durch die Verwendung von Magnesiumorotat können die Prävention und Therapie des metabolischen Syndroms noch einmal deutlich gesteigert werden.

Die Übertragbarkeit der obigen, aus Tierexperimenten stammenden Ergebnisse auf den Menschen ergibt sich aus Einzelfallbeobachtungen an Patienten, an denen sich die oben beschriebenen Wirkungen von Magnesiumorotat zeigten.

## Patentansprüche

1. Verwendung von Orotsäure zur Herstellung eines Arzneimittels zur Prävention des metabolischen Syndroms.

2. Verwendung von Orotsäure zur Herstellung eines Arzneimittels zur Therapie des metabolischen Syndroms.

3. Verwendung nach Anspruch 1 oder Anspruch 2, dadurch gekennzeichnet, daß ein Salz der Orotsäure verwendet wird.

4. Verwendung nach Anspruch 3, dadurch gekennzeichnet, daß Magnesiumorotat verwendet wird.

5. Verwendung nach einem der Ansprüche 1 bis 4, dadurch gekennzeichnet, daß eine Mischung aus Orotsäure und einer Magnesiumverbindung verwendet wird.

6. Verwendung nach einem der Ansprüche 1 bis 5, dadurch gekennzeichnet, daß eine Mischung aus einem Salz der Orotsäure und einer Magnesiumverbindung verwendet wird.

7. Verwendung nach Anspruch 5 oder 6, dadurch gekennzeichnet, daß eine Mischung aus Orotsäure und Magnesiumorotat verwendet wird.
